# EUROPÄISCHE PATENTANMELDUNG

(11) **EP 4 686 444 A1**
(43) Veröffentlichungstag der Anmeldung: **04.02.2026**
(21) Anmeldenummer: 24192141.0
(22) Anmeldetag: 31.07.2024
(51) Int. Cl.: A61B 3/00, A61B 3/032, A61B 3/113

(54) **BESTIMMUNG EINES NEURONALEN GESUNDHEITSZUSTANDS EINER PERSON**

(71) Anmelder: ETM professional control GmbH, 7000 Eisenstadt (AT)
(72) Erfinder: Hoegerl, Daniel, 7053 Hornstein (AT)
(74) Vertreter: Siemens Patent Attorneys

(57) **Zusammenfassung**

Eine Vorrichtung (100) zur Bestimmung eines neurologischen Gesundheitszustands einer Person (105) umfasst eine optische Anzeige (110) zur Bereitstellung eines optischen Reizes (115) an die Person (105); eine Abtasteinrichtung zur Bestimmung einer Blickrichtung (120) der Person (105) bezüglich des optischen Reizes (115); eine Verarbeitungseinrichtung (135), die dazu eingerichtet ist, auf der Basis der erkannten Blickrichtung (120) und eines bereitgestellten optischen Reizes (115) eine neurologische Schwäche der Person (105) zu erkennen; und eine Ausgabevorrichtung zur Bereitstellung eines Bestimmungsergebnisses.

## Beschreibung

Die vorliegende Erfindung betrifft die Bestimmung eines neuronalen Gesundheitszustands einer Person. Insbesondere betrifft die Erfindung die Bestimmung des Gesundheitszustands auf der Basis einer Augenuntersuchung.

Es ist bekannt, dass eine Anzahl von Krankheiten, unter der eine Person leiden kann, mit okulomotorischen Störungen einhergeht. Solche Störungen betreffen die Steuerung der Augen der Person und gehen auf eine neurologische Schwäche zurück, die mittels einer geeigneten Augenuntersuchung bestimmt werden kann. Dabei wird die Person üblicherweise gebeten, auf einen vorbestimmten optischen Reiz auf eine angegebene Weise zu reagieren. Beispielsweise kann die Person einen vorbestimmten Punkt mit den Augen fixieren oder eine sich bewegende Marke mit den Augen verfolgen. Verhält sich die Blickrichtung bezüglich des optischen Reizes auffällig, so kann auf das Vorliegen einer neurologischen Störung geschlossen werden.

Eine solche Augenuntersuchung ist üblicherweise zeitintensiv und benötigt geeignete Einrichtungen und zur Durchführung geschultes Personal. Die Bewertung einer festgestellten Blickrichtung, um auf eine mögliche Erkrankung der Person zu schließen, kann schwierig sein und erfordert im Allgemeinen die Mitarbeit eines Experten, insbesondere eines Arztes. Regelmäßige Augenuntersuchungen zur Feststellung eines neurologischen Gesundheitszustands sind daher nicht üblich.

Eine der vorliegenden Erfindung zu Grunde liegende Aufgabe besteht in der Bereitstellung einer verbesserten Technik zur möglichst einfachen und sicheren Bestimmung eines neurologischen Gesundheitszustands einer Person. Die Erfindung löst diese Aufgabe mittels der Gegenstände der unabhängigen Ansprüche. Unteransprüche geben bevorzugte Ausführungsformen wieder.

Nach einem ersten Aspekt der vorliegenden Erfindung umfasst eine Vorrichtung zur Bestimmung eines neurologischen Gesundheitszustands einer Person eine optische Anzeige zur Bereitstellung eines optischen Reizes an die Person; eine Abtasteinrichtung zur Bestimmung einer Blickrichtung der Person bezüglich des optischen Reizes; eine Verarbeitungseinrichtung, die dazu eingerichtet ist, auf der Basis der erkannten Blickrichtung und eines bereitgestellten optischen Reizes eine neurologische Schwäche der Person zu erkennen; und eine Ausgabevorrichtung zur Bereitstellung eines Bestimmungsergebnisses.

Im Unterschied zu bekannten Techniken muss kein vorbestimmter optische Reiz bereitgestellt werden, vielmehr kann ein beliebiger anderweitig erzeugter optischer Reiz ausgewertet werden. Die Vorrichtung kann keine besonderen Untersuchungsinstrumente umfassen und kostengünstig hergestellt und betrieben werden. Dabei kann die Vorrichtung die Bestimmung weitgehend oder vollständig automatisch durchführen, der Einsatz von Personal ist zur Durchführung der Untersuchung nicht erforderlich. Der neurologische Gesundheitszustand der Person kann mit einfachen Mitteln rasch und mit wenig Aufwand bestimmt werden. So können auch regelmäßige Untersuchungen mit geringem Aufwand durchgeführt werden.

Nach einem weiteren Aspekt der vorliegenden Erfindung kann eine Vorrichtung zur Bestimmung einer Blickrichtung einer Person eine optische Anzeige zur Bereitstellung eines optischen Reizes an die Person; eine Abtasteinrichtung zur Bestimmung einer Blickrichtung der Person bezüglich des optischen Reizes; eine Verarbeitungseinrichtung, die dazu eingerichtet ist, auf der Basis der erkannten Blickrichtung und eines bereitgestellten optischen Reizes einen Blickparameter zu bestimmen; und eine Ausgabevorrichtung zur Bereitstellung eines Bestimmungsergebnisses umfassen. In einer Ausgestaltung der Erfindung kann der Blickparameter einen Zusammenhang zwischen dem optischen Reiz und der Blickrichtung abbilden oder eine Antwort des Auges mittels der Blickrichtung auf einen optischen Reiz darstellen. In einer bevorzugten Ausgestaltung der Erfindung kann aus dem Blickparameter eine neurologische Schwäche der Person abgeleitet oder bestimmt werden. Als Bestimmungsergebnis kann der Blickparameter ausgegeben werden. Der Blickparameter kann als, insbesondere reiner, Messwert verstanden werden und von einer medizinisch geschulten Person anschließend interpretiert werden.

Bevorzugt ist die Verarbeitungseinrichtung dazu eingerichtet, mehrere vorbestimmte Merkmale der Blickrichtung bezüglich des optischen Reizes zu bestimmen. Fokussiert die Person beispielsweise auf einen unbeweglichen Punkt der Darstellung, so kann eine fortlaufende Augenbewegung erfasst werden, die auch Augenzittern oder Nystagmus genannt werden kann. Konvergieren Blickrichtungen eines rechten und eines linken Auges nicht vollständig miteinander, so kann auf eine Fehlstellung der Augen geschlossen werden. Bewegt sich ein Auge nur schleppend oder mit einer anderen Geschwindigkeit als das andere Auge, so kann eine Amblyopie erkannt werden, die auch träges Auge genannt wird. Bewegt sich die Blickrichtung über den optischen Reiz hinweg, so kann ein Überschießen oder Unterschießen der Blickrichtung bei bestimmten Augenbewegungen erfasst werden. Auch eine mechanische Beschränkung der Beweglichkeit eines Auges kann erfasst werden. Die bestimmten Merkmale können als Parameter ausgewertet werden, um auf das Vorliegen einer Auffälligkeit bzw. Anomalie zu schließen.

Es wird vorgeschlagen, einen bestehenden optischen Reiz auf die Person zu erfassen und ihre Reaktion in Form einer Blickrichtung bezüglich des erkannten optischen Reizes zu analysieren. Der Reiz kann von einer externen Einrichtung an der Anzeige hervorgerufen werden. Es wurde erkannt, dass mit modernen Methoden der Bildverarbeitung mit guter Genauigkeit bestimmt werden kann, ob eine erfasste Augenbewegung bezüglich eines erfassten optischen Reizes auffällig oder unauffällig ist. Streng formale oder künstliche optische Reize sind für diese Bestimmung nicht erforderlich.

Es ist besonders bevorzugt, dass die Verarbeitungseinrichtung dazu eingerichtet ist, den externen optischen Reiz zu klassifizieren und die Bestimmung durchzuführen, wenn der Reiz in eine vorbestimmte Klasse fällt. So kann ein optischer Reiz, der keine diagnostischen Erkenntnisse verspricht, für die Bestimmung der Augenbewegung vernachlässigt werden. Ein anderer optischer Reiz, der eine Bestimmungen eines oder mehrerer vorbestimmter Merkmale erlaubt, kann ausgenutzt werden, um aussagekräftige Daten für die Bestimmung zu sammeln. Unterschiedliche Klassen können beispielsweise gebildet sein für einen Text, eine Grafik, ein Fixierbild oder eine neutrale grafische Darstellung, die beispielsweise mit einer akustischen Darbietung an die Person einhergehen kann. Die Klassifikation kann umfassen, dass der Reiz über eine vorbestimmte Zeit Anforderungen einer Klasse erfüllt. Auch eine Komplexität des optischen Reizes kann berücksichtigt werden. Die Komplexität des Reizes sollte üblicherweise nicht zu hoch, aber auch nicht zu niedrig sein.

In einer besonders bevorzugten Ausführungsform umfasst die Anzeige einen Bildschirm und der optische Reiz eine Ausgabe einer vorbestimmten Computeranwendung. Die Computeranwendung kann den Bildschirm als Ausgabemedium verbinden. Beispielhafte Computeranwendungen, die für die vorgestellte Technik geeignet sein können, umfassen eine Anwendung zum Anzeigen oder Editieren von Text, für elektronische Post (E- Mail), für HTML-Seiten oder eine vorbestimmte Büroanwendung. Die vorbestimmte Büroanwendung kann vorbestimmte Interaktionen ermöglichen, beispielsweise das Anzeigen eines ausgewählten Inhalts oder das Blättern durch solche Inhalte.

Durch die Nutzbarkeit optischer Reize einer solchen Computeranwendung kann der neurologische Gesundheitszustand der Person bestimmt werden, während die Person üblichen Tätigkeiten an einem Computer mit Bildschirm nachgeht. Insbesondere kann der Gesundheitszustand bestimmt werden, während die Person arbeitet oder einer anderen Tätigkeit am Computer nachgeht. Es wurde erkannt, dass bei üblichen Tätigkeiten an einem Computer regelmäßig optische Reize anfallen, die zur Bestimmung des neurologischen Gesundheitszustands der Person ausgenutzt werden können.

Beispielsweise kann die Vorrichtung an einem Arbeitsrechner eingesetzt werden, sodass der Gesundheitszustand der Person regelmäßig oder auch fortlaufend überwacht werden kann. Eine sich anbahnende Krankheit, die mit einer neurologischen Auffälligkeit im Augenbereich einhergeht, kann dadurch schon frühzeitig erkannt werden. Da eine Vielzahl Krankheiten mit hierin beschriebenen neurologischen Anomalien einhergehen, kann eine allgemeine Aussage über den Gesundheitszustand der Person getroffen werden. Sollte sich zeigen, dass Grund zur Sorge für eine Erkrankung vorliegt, so kann eine entsprechende Untersuchung an der Person vorgenommen werden, um eine mögliche Ursache in Form einer Krankheit für den festgestellten Gesundheitszustand zu bestimmen. Eine solche Untersuchung erfordert üblicherweise eine eingehende Untersuchung durch einen Arzt.

Weiter bevorzugt realisiert die Verarbeitungseinrichtung eine Methode des maschinellen Lernens und ist darauf trainiert, auf der Basis der erkannten Blickrichtung und des bereitgestellten optischen Reizes eine vorbestimmte neurologische Schwäche zu erkennen.

Insbesondere kann die Verarbeitungseinrichtung darauf trainiert werden, eine Anzahl vorbestimmter neurologischer Schwächen zu erkennen. Dazu können einzeln festgestellte Merkmale der Blickrichtung bezüglich des optischen Reizes ausgewertet werden. Unterschiedlich stark ausgeprägten Merkmalen kann nach Art eines Fingerabdrucks eine neurologische Schwäche zugeordnet werden, sodass eine mögliche Erkrankung der Person eingegrenzt werden kann.

In einer bevorzugten Ausführungsform umfasst die Methode des maschinellen Lernens einen Entscheidungsbaum, der auch als Decision Tree bekannt ist. Bei dieser Methode kann die Ausprägung unterschiedlicher Merkmale in einer zufälligen oder pseudozufälligen Reihenfolge nach Art eines Entscheidungsbaums ausgewertet werden. Im Lauf des Trainings können Schwellenwerte für die einzelnen Entscheidungen innerhalb des Baums gefunden werden. Bereits diese einfache Technik des maschinellen Lernens hat zu guten Ergebnissen bei der Bestimmung neurologischer Gesundheitszustände geführt.

In einer Weiterentwicklung der Erfindung kann die Methode des maschinellen Lernens einen Zufallswald umfassen, der auch als Random Forest bekannt ist. Dabei können mehrere Entscheidungsbäume aufgestellt werden, die mit zufallsbestimmten, unterschiedlichen Wurzelelementen beginnen. Als Ergebnis kann ein Mittelwert der Entscheidungen mehrerer Entscheidungsbäume verwendet werden. Ausreißer oder krasse Fehlbestimmungen können so erfolgreich unterdrückt werden.

Weiter bevorzugt umfasst die Abtasteinrichtung eine Kamera zur Abtastung eines Auges der Person. Die Kamera kann an der Anzeige bzw. dem Bildschirm befestigt sein. In einer Ausführungsform ist die Kamera mit dem Bildschirm integriert ausgeführt. Eine solche Ausführungsform ist aktuell an vielen gewerblich genutzten Computern oder Bürocomputern zu finden. Die vorgestellte Technik kann dadurch verbessert flächendeckend eingesetzt werden, ohne große zusätzliche Kosten zu verursachen.

In einer anderen Ausführungsform kann die Abtasteinrichtung auch eine Kamera umfassen, die am Kopf der Person befestigt ist, während die Anzeige ortsfest ist. In dieser Ausführungsform ist es jedoch erforderlich, dass die Person eine vorbestimmte Kopfhaltung einnimmt oder die Kopfhaltung zusätzlich bestimmt wird. Eine am Kopf getragene Abtasteinrichtung kann eine genauere Auswertung der Blickrichtung ermöglichen.

In noch einer weiteren Ausführungsform ist sowohl die Abtasteinrichtung als auch die Anzeige am Kopf der Person befestigt. Beispielsweise können die Anzeige und die Abtasteinrichtung nach Art einer VR-Brille miteinander integriert ausgeführt sein. In diesem Fall kann die Bestimmung einer Ausrichtung des Kopfes der Person entfallen, obwohl gängige VR-Brillen eine derartige Bestimmung durchführen und ein Bestimmungsergebnis zur Verfügung stellen.

Es wird weiterhin vorgeschlagen, dass die hierin beschriebene Vorrichtung derart aufgebaut und implementiert ist, dass Persönlichkeitsrechte der Person keinesfalls verletzt werden. Insbesondere soll die Erhebung und Verarbeitung personenbezogener Daten der Kontrolle der Person unterliegen. Ein Ergebnis einer Bestimmung soll nur weitergegeben werden, falls die Person dem ausdrücklich zustimmt.

Die Vorrichtung kann eine Ausgabeeinrichtung an die Person umfassen, wobei die Vorrichtung dazu eingerichtet ist, einen Hinweis auf eine Bestimmung des Gesundheitszustands bereitzustellen. Der Hinweis kann bereitgestellt werden, wenn die Bestimmung gerade durchgeführt wird oder unmittelbar bevorsteht. Die Ausgabeeinrichtung kann beispielsweise optisch, akustisch oder auch haptisch umgesetzt sein. In einer Ausführungsform ist die optische Ausgabeeinrichtung mit der Anzeige integriert ausgeführt. In einer anderen Ausführungsform kann die Ausgabeeinrichtung ein separates Gerät umfassen, beispielsweise eine Signalleuchte, die im Bereich der Anzeige angebracht werden kann.

Die Vorrichtung kann eine Ausgabeeinrichtung an die Person umfassen, wobei die Vorrichtung dazu eingerichtet ist, einen Hinweis auf eine Bestimmung des Blickparameters bereitzustellen.

Es ist weiterhin bevorzugt, dass die Vorrichtung eine Eingabeeinrichtung für die Person umfasst, wobei die Verarbeitungseinrichtung dazu eingerichtet ist, ein Einverständnis der Person zur Durchführung einer Bestimmung ihres Gesundheitszustands zu erfassen. Die Eingabeeinrichtung kann eine übliche Einrichtung sein, die an einem Computer verwendet werden kann, beispielsweise eine Tastatur, eine Computermaus oder ein digitales Tablett. Andere Eingabemethoden, beispielsweise durch Gestensteuerung, sind ebenfalls möglich. Ein Einverständnis der Person kann auf eine unmittelbar bevorstehende oder gerade durchgeführte Bestimmung bezogen sein oder eine generelle Erlaubnis betreffen, eine entsprechende Erhebung durchzuführen. Eine Weiterleitung oder Auswertung eines bestimmten neurologischen Gesundheitszustands kann eine weitere, dedizierte Zustimmung der Person erfordern. Dies kann sowohl gelten, wenn eine neurologische Auffälligkeit bestimmt wurde, oder die Bestimmung darauf schließen lässt, dass die Person neurologisch gesund ist.

Die Vorrichtung kann teilweise oder vollständig mit einem bekannten Computer integriert ausgeführt sein, wobei entscheidende Schritte der Verarbeitung auf einer Verarbeitungseinrichtung des Computers ausgeführt werden können. Dabei ist dafür Sorge zu tragen, dass die Verarbeitung nach den geltenden Regeln des Datenschutzes erfolgt und keine personenbezogenen Daten, insbesondere keine Gesundheitsdaten, auf unzulässige Weise verarbeitet oder geteilt werden.

Es ist weiterhin bevorzugt, dass die Vorrichtung eine Eingabeeinrichtung für die Person umfasst, wobei die Verarbeitungseinrichtung dazu eingerichtet ist, ein Einverständnis der Person zur Durchführung einer Bestimmung des Blickparameters zu erfassen.

Nach einem weiteren Aspekt der vorliegenden Erfindung umfasst ein erstes Verfahren zum Bestimmen eines neurologischen Gesundheitszustands einer Person Schritte des Erfassens eines der Person dargebotenen optischen Reizes; des Bestimmens einer Blickrichtung der Person bezüglich des optischen Reizes; des Erkennens einer neurologischen Schwäche der Person auf der Basis der erkannten Blickrichtung und des bereitgestellten optischen Reizes; und des Bereitstellens eines Bestimmungsergebnisses.

Dabei wird der optische Reiz bevorzugt nicht generiert oder anderweitig kontrolliert, sondern ein bestehender optischer Reiz wird durch die Verarbeitungseinrichtung ausgewertet.

Das Verfahren kann teilweise oder vollständig mittels einer hierin beschriebenen Vorrichtung ausgeführt werden. Dazu kann die Vorrichtung eine insbesondere elektronisch ausgeführte Verarbeitungseinrichtung umfassen, die etwa als integrierter Schaltkreis, als programmierbarer Logikbaustein oder als programmierbarer Mikrocomputer ausgebildet ist. Das Verfahren kann in Form einer Konfiguration oder als Computerprogrammprodukt mit Programmcodemitteln für die Verarbeitungseinrichtung realisiert sein. Die Konfiguration bzw. das Computerprogrammprodukt kann auf einem computerlesbaren Datenträger abgespeichert sein. Merkmale oder Vorteile des ersten Verfahrens können auf die Vorrichtung übertragen werden oder umgekehrt.

Nach einem weiteren Aspekt der vorliegenden Erfindung umfasst ein weiteres Verfahren zum Bestimmen einer Blickrichtung einer Person Schritte des Erfassens eines der Person dargebotenen optischen Reizes; des Bestimmens einer Blickrichtung der Person bezüglich des optischen Reizes; des Ermittelns eines Blickparameters auf der Basis der erkannten Blickrichtung und des bereitgestellten optischen Reizes; und des Bereitstellens eines Bestimmungsergebnisses, bevorzugt in der Form des Blickparameters.

Nach noch einem weiteren Aspekt der vorliegenden Erfindung umfasst ein zweites Verfahren zum Trainieren einer Verarbeitungseinrichtung, die eine Technik des maschinellen Lernens realisiert, zur Bestimmung eines neurologischen Gesundheitszustands einer Person, Schritte des Erfassens eines neurologischen Gesundheitszustands einer Referenzperson; des Verfolgens einer Blickrichtung der Referenzperson auf einer Anzeige, auf welcher der Referenzperson ein vorbestimmter optischer Reiz dargeboten ist; des Trainierens der Verarbeitungseinrichtung zur Erkennung des erfassten neurologischen Gesundheitszustands auf der Basis der Blickrichtung bzw. deren Verlaufs; wobei die genannten Schritte an einer Vielzahl Referenzpersonen durchgeführt werden.

An einer Referenzperson kann eine Vielzahl optischer Reize mit jeweils zugeordneten Blickrichtungen ausgewertet werden. Es ist zu beachten, dass optische Reize unter den Referenzpersonen nicht notwendigerweise vergleichbar sein müssen. Allerdings ist bevorzugt, dass die optischen Reize in eine vorbestimmte Klasse fallen. Dazu können die optischen Reize klassifiziert werden, und die Auswertung der Blickrichtung der Referenzperson kann nur dann erfolgen, wenn die optischen Reize in eine vorbestimmte Klasse fallen.

Das zweite Verfahren kann dazu verwendet werden, eine hierin beschriebene Vorrichtung zu erstellen bzw. zu trainieren oder zu konfigurieren. Das zweite Verfahren kann mittels der hierin beschriebenen Vorrichtung oder mittels einer anderen Vorrichtung ausgeführt werden. Die Bestimmung des neurologischen Gesundheitszustands der Referenzpersonen erfolgt bevorzugt mittels anderer Methoden als der hierin beschriebenen Blickrichtungsüberwachung. Insbesondere kann der Gesundheitszustand einer Referenzperson durch einen Arzt diagnostisch festgestellt werden.

Es ist besonders bevorzugt, dass die Referenzpersonen erste Personen umfassen, die neurologisch unauffällig sind, und zweite Personen, die an einer neurologischen Schwäche leiden. So kann die Verarbeitungseinrichtung darauf trainiert werden, sowohl neurologisch unauffällige als auch neurologisch auffällige Personen anhand der beschriebenen Blickrichtungsauswertung zu erkennen. Sollen unterschiedliche neurologische Schwächen unterschieden werden, so kann für jede Schwäche eine vorbestimmte Anzahl Referenzpersonen mit der jeweiligen Schwäche ausgewertet werden. Eine Anzahl erster Personen ohne neurologische Auffälligkeiten kann dabei ähnlich groß sein.

Nach noch einem weiteren Aspekt der vorliegenden Erfindung umfasst ein weiteres Verfahren zum Trainieren einer Verarbeitungseinrichtung, die eine Technik des maschinellen Lernens realisiert, zur Bestimmung eines Blickparameters einer Person, Schritte des Erfassens eines Blickparameters einer Referenzperson; des Verfolgens einer Blickrichtung der Referenzperson auf einer Anzeige, auf welcher der Referenzperson ein vorbestimmter optischer Reiz dargeboten ist; des Trainierens der Verarbeitungseinrichtung zum Ermitteln eines Blickparameters auf der Basis der Blickrichtung bzw. deren Verlaufs; wobei die genannten Schritte an einer Vielzahl Referenzpersonen durchgeführt werden.

Die Erfindung wird nun mit Bezug auf die beigefügten Figuren genauer beschrieben, wobei
- Figur 1: eine Vorrichtung;
- Figur 2: ein System;
- Figur 3: ein Ablaufdiagramm eines ersten Verfahrens; und
- Figur 4: ein Ablaufdiagramm eines zweiten Verfahrens
darstellt.

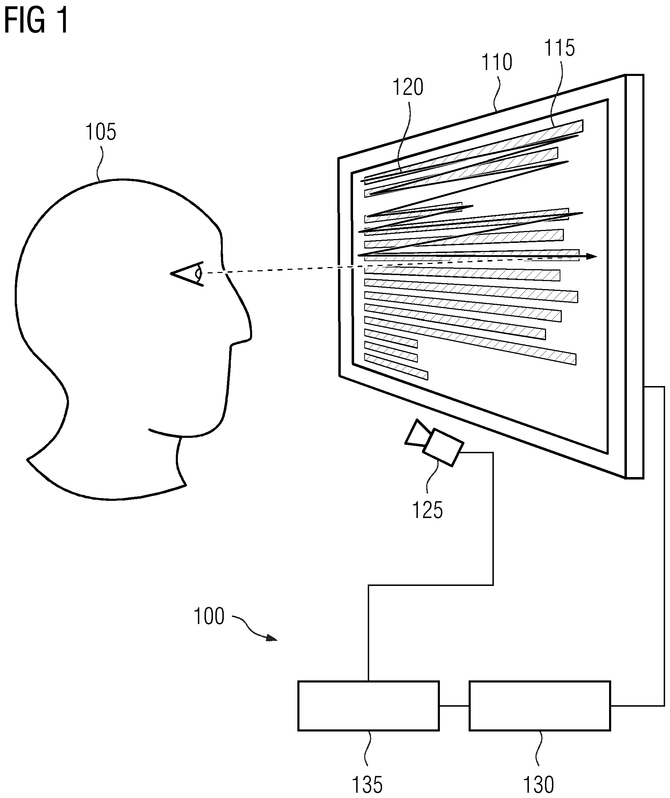

Figur 1 zeigt eine Vorrichtung 100 zur Bestimmung eines neurologischen Gesundheitszustands einer Person 105. Mittels einer Anzeige 110 können der Person 105 optische Reize 115 dargeboten werden. Die Person 105 kann einen optischen Reiz 115 mit ihren Augen abtasten, wobei sie in der Regel eine Blickrichtung 120 ändert. In der Darstellung von Figur 1 ist die Anzeige 110 als Monitor eines Computers dargestellt, und der optische Reiz 115 umfasst ebenfalls exemplarisch einen Text. Die beispielhaft dargestellte Blickrichtung 120 verläuft in Leserichtung über den Text 115, wobei nicht notwendigerweise alle Zeilen in der dafür vorgesehenen Reihenfolge abgetastet werden.

Die Vorrichtung 100 umfasst eine Abtasteinrichtung 125, die dazu eingerichtet ist, die Blickrichtung 120 der Person 105 zu bestimmen. Die Abtasteinrichtung 125 kann insbesondere eine Kamera umfassen, die auf die Person 105 und insbesondere ihre Augen gerichtet ist. Eine Ausrichtung der Kamera 125 bezüglich der Anzeige 110 kann fest sein. Insbesondere kann die Kamera 125 fest an der Anzeige 110 installiert sein, beispielsweise wie dargestellt unterhalb der Anzeige 110 oder auch in einem oberen Bereich. Eine separate Ausführungsform ist ebenfalls möglich.

In einer anderen Ausführungsform kann die Abtasteinrichtung 125 am Kopf der Person 105 getragen werden. Eine Ausrichtung des Kopfs kann mittels derselben oder einer anderen Abtasteinrichtung 125 bestimmt werden. So kann einerseits die Lage der Abtasteinrichtung 125 bezüglich des optischen Reizes 115 und andererseits die Blickrichtung 120 bezüglich der Abtasteinrichtung 125 bestimmt werden. Im Ergebnis lässt sich so die dargestellte Blickrichtung 120 bezüglich des Reizes 115 nachvollziehen.

Der optische Reiz 115 kann durch einen üblichen Computer 130 bereitgestellt werden. Dabei ist bevorzugt, dass die Person 105 mit dem Computer 130 interagieren kann. Insbesondere kann die Person 105 die Möglichkeit haben, eine Eingabe an den Computer 130 bereitzustellen, beispielsweise über eine Tastatur oder eine Computermaus. Eine Funktion des Computers 130 und insbesondere ein bereitgestellter optischer Reiz 115 kann in Abhängigkeit der Eingabe bestimmt werden. Eine auf dem Computer 130 ablaufende Applikation, welche den optischen Reiz 115 auf der Anzeige 110 generiert, kann eine beliebige, insbesondere interaktive, Anwendung umfassen. Die Anwendung kann beispielsweise einen Text, Bilder oder auch eine Benutzeroberfläche auf der Anzeige 110 darstellen. Die Person 105 kann die Applikation mittels einer entsprechenden Eingabeeinrichtung steuern.

Eine Verarbeitungseinrichtung 135, die eigenständig oder integriert mit dem Computer 130 ausgeführt sein kann, empfängt den optischen Reiz 115 und die mittels der Abtasteinrichtung 125 bestimmte Blickrichtung 120 der Person 105. Dabei kann die Verarbeitungseinrichtung 135 auf der Basis eines Kamerabilds der Verarbeitungseinrichtung 135 oder anderer zur Verfügung gestellter Daten die Blickrichtung 120 bestimmen. Der optische Reiz 115 kann als elektronisches Signal oder als entsprechende Information vom Computer 130 übernommen werden. Eine Repräsentation des optischen Reizes 115 kann beispielsweise als Videosignal oder als digital repräsentierter Bildschirminhalt übermittelt werden. In einer anderen, nicht dargestellten Ausführungsform ist eine weitere Kamera vorgesehen, um den optischen Reiz 115 auf der Anzeige 110 zu erfassen. Eine solche Kamera würde typischerweise hinter oder neben der Person 105 mit Erfassungsrichtung zur Anzeige 110 angebracht werden.

Die Verarbeitungseinrichtung 135 ist dazu eingerichtet, auf der Basis des erfassten optischen Reizes 115 und der erfassten Blickrichtung 120 zu bestimmen, ob eine neurologische Schwäche der Person 105 im optischen Wahrnehmungsapparat erkennbar ist. Auf dieser Basis kann ein neurologischer Gesundheitszustand der Person 105 erkannt werden. Ein Bestimmungsergebnis kann beispielsweise mittels der Anzeige 110 bereitgestellt werden. Andere Möglichkeiten umfassen eine dedizierte Ausgabevorrichtung, eine Datei oder ein Nachrichtensignal.

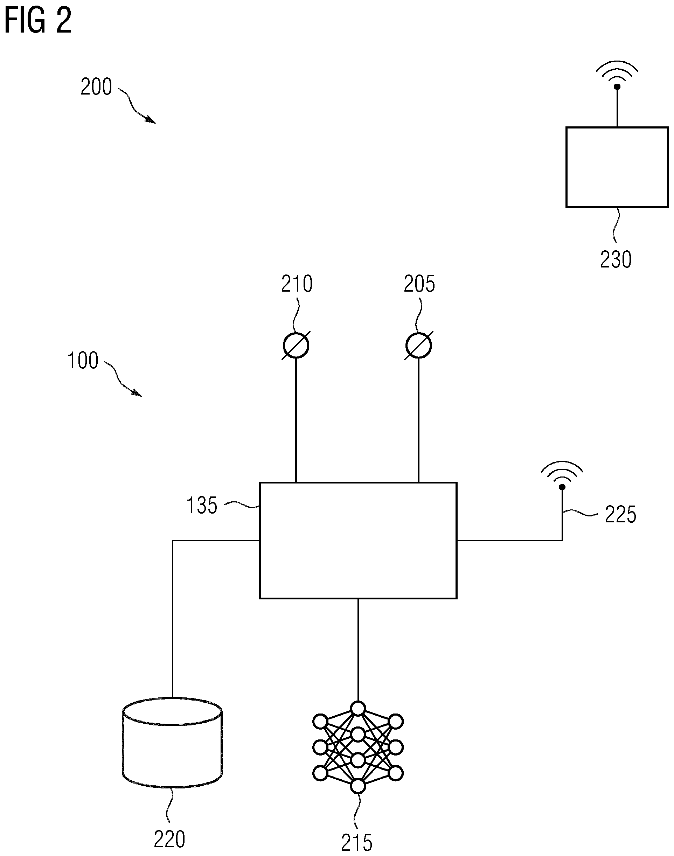

Figur 2 zeigt ein System 200 mit einer Vorrichtung 100. Die Verarbeitungseinrichtung 135 der Vorrichtung 100 ist mit einer ersten Schnittstelle 205 und einer zweiten Schnittstelle 210 verbunden. Über die erste Schnittstelle 205 empfängt die Verarbeitungseinrichtung 135 einen optischen Reiz 115, welche der Person 105 dargeboten ist, in einer beliebigen Repräsentation bzw. Form. Über die zweite Schnittstelle 210 empfängt die Verarbeitungseinrichtung 135 Informationen über die Blickrichtung 120 der Person 105.

Die Verarbeitungseinrichtung 135 umfasst oder ist verbunden mit einer Einrichtung 215, die maschinell lernfähig ist. In symbolischer Form ist die Einrichtung 215 als künstliches neuronales Netzwerk (KNN) dargestellt. Es sind jedoch auch andere Techniken möglich, mit denen die Einrichtung 215 anhand von Exempeln dazu trainiert werden kann, eine vorbestimmte Aufgabe durchzuführen.

Rein optional ist ein Datenspeicher 220 vorgesehen, in welchem Messwerte abgelegt werden können. Solche Messwerte können insbesondere einen erfassten optischen Reiz 115 und eine dazu korrespondierende Blickrichtung 120 einer Person 105 umfassen. Das Zwischenspeichern solcher Werte kann insbesondere beim Trainieren der Einrichtung 215 erforderlich sein.

Die dargestellte Vorrichtung 100 kann dazu verwendet werden, die Einrichtung 215 zum maschinellen Lernen zu trainieren. In einer anderen Ausführungsform kann über eine dritte Schnittstelle 225 eine externe Einrichtung 230 angebunden werden. Die dritte Schnittstelle 225 ist exemplarisch als drahtlose Schnittstelle dargestellt, kann jedoch auch eine beliebige andere Schnittstelle umfassen. Die externe Einrichtung 230 kann zu einem Zeitpunkt oder nacheinander mit verschiedenen Vorrichtungen 100 verbunden sein. Eine Vorrichtung 100 kann dazu verwendet werden, optische Reize 115 und jeweils zugeordnete Blickrichtungen 120 einer Person 105 zu erfassen und an die externe Einrichtung 230 zu übermitteln. Bezüglich der Person 105 kann bekannt sein, ob sie unter einer neurologischen Schwäche leidet bzw. ob ihr neurologischer Gesundheitszustand auffällig oder unauffällig ist. Auf der Basis einer möglichst großen Vielzahl solcher Daten kann durch die externe Einrichtung 230 eine korrespondierende Einrichtung 215 trainiert und anschließend an eine Vorrichtung 100 übermittelt werden. Typischerweise ist für das Durchführen eines Trainings eine hohe Verarbeitungsleistung erforderlich. Dabei können sehr viele Datensätze verarbeitet werden. Ein trainiertes Modell bzw. eine trainierte Einrichtung 215 zum maschinellen Lernen ist hingegen üblicherweise von geringem Umfang und kann leicht an eine oder mehrere Vorrichtungen 100 übermittelt werden. Das Durchführen der hierin beschriebenen Bestimmungsaufgabe mittels der trainierten Einrichtung 215 kann wenig aufwendig sein.

Anschließend kann eine Vorrichtung 100 dazu verwendet werden, den neurologischen Gesundheitszustand einer weiteren Person 105 auf der Basis einer Kombination eines dargebotenen optischen Reizes 115 und einer korrespondierenden Blickrichtung 120 zu bestimmen.

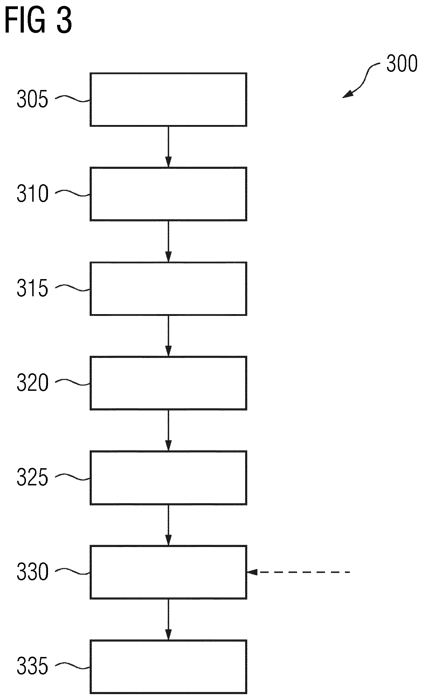

Figur 3 zeigt ein Ablaufdiagramm eines ersten Verfahrens 300 zum Bestimmen eines neurologischen Gesundheitszustands einer Person 105. Das Verfahren 300 kann insbesondere auf einer Vorrichtung 100 ablaufen.

In einem Schritt 305 kann ein Benutzereinverständnis der Person 105 zum Bestimmen ihres neurologischen Gesundheitszustands eingeholt werden. Dazu kann insbesondere eine entsprechende Eingabe der Person 105 erfasst werden. Wird eine Bestimmung durchgeführt bzw. wird eine Blickrichtung 120 der Person 105 bezüglich eines optischen Reizes 115 erfasst, so kann ein entsprechender Hinweis an die Person 105 bereitgestellt werden. Der Hinweis kann beispielsweise optisch, akustisch oder auch haptisch bereitgestellt sein.

In einem Schritt 310 kann der optische Reiz 115 erfasst werden. Dazu kann eine auf der Anzeige 110 bereitgestellte optische Darstellung erfasst werden. In einem Schritt 315 kann der Reiz klassifiziert werden. Insbesondere kann bestimmt werden, ob der optische Reiz 115 dazu geeignet ist, der Bestimmung einer neurologischen Schwäche der Person 105 zu Grunde gelegt zu werden. Handelt es sich bei dem optischen Reiz 115 beispielsweise um eine komplexe Darstellung in Form eines Films oder einer bewegten Grafik mit vielen Elementen, so kann der optische Reiz 115 verworfen werden. Liegt andererseits ein gut kontrollierter optischer Reiz 115 vor, beispielsweise ein zu lesender Text, so kann der optische Reiz 115 als verarbeitbar klassifiziert werden. Optische Reize 115 können auch in unterschiedliche Klassen unterteilt werden, wobei insbesondere unterschiedliche Klassen verarbeitbarer Reize vorgesehen sein können. Die optischen Reize 115 unterschiedlicher Klassen können auf unterschiedliche Merkmale des Zusammenhangs der Blickrichtung 120 mit dem optischen Reiz 115 hinweisen. Unterschiedliche Kategorien können auch für unterschiedliche vorbestimmte neurologische Schwächen gebildet sein.

Bezüglich eines verarbeitbaren optischen Reizes 115 kann in einem Schritt 320 eine Augenbewegung der Person 105 erfasst werden. Auf der Basis der Augenbewegung und evtl. noch weiterer Informationen kann die Blickrichtung 120 bezüglich der Anzeige 110 bestimmt werden.

In einem Schritt 325 kann bezüglich eines optischen Reizes 115 und einer dazu korrespondierenden Blickrichtung 120 der Person 105 ein neurologisches Merkmal bestimmt werden. Beispielhafte neurologische Merkmale umfassen ein Augenzittern, eine Fehlstellung der Augen, ein träges Auge, ein Über- oder Unterschießen bei bestimmten Augenbewegungen oder eine mechanische Beschränkung der Augenbewegung. Weitere Merkmale sind ebenfalls möglich.

In einem Schritt 330 können die bestimmten neurologischen Merkmale mittels einer Technik des maschinellen Lernens verwendet werden, um einen neurologischen Gesundheitszustand der Person 105 zu bestimmen. Der Gesundheitszustand kann in einer einfachen Ausführungsform entweder darauf hinweisen, dass die Person 105 möglicherweise unter einer neurologischen Schwäche leidet oder als symptomfrei gelten kann. In einer weitergeführten Ausführungsform können unterschiedliche Anomalien oder Auffälligkeiten ausgewertet werden, die jeweils auf eine bestimmte neurologische Schwäche der Person 105 hinweisen.

Ein Hinweis auf ein Bestimmungsergebnis kann in einem Schritt 335 bereitgestellt werden. Der Hinweis kann beispielsweise in elektronischer oder in menschenleserlicher Form ergehen und ist bevorzugt zunächst nur an die Person 105 gerichtet.

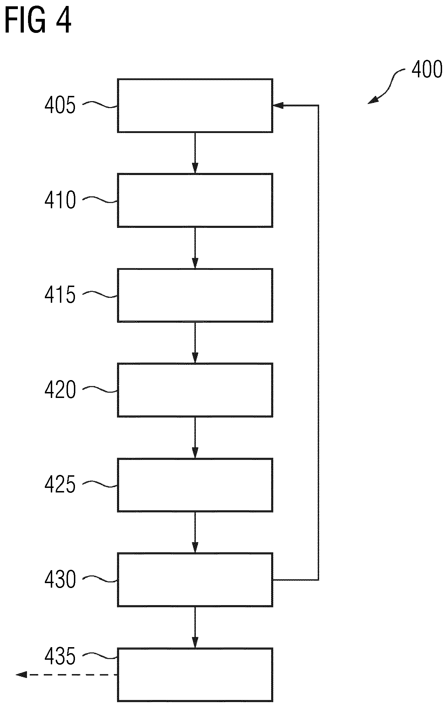

Figur 4 zeigt ein Ablaufdiagramm eines zweiten Verfahrens 400, welches mittels einer Vorrichtung 100 oder eines Systems 200 ausgeführt werden kann. Das zweite Verfahren 400 dient dazu, eine Einrichtung 215 zum maschinellen Lernen derart zu trainieren, dass sie im Rahmen des ersten Verfahrens 300 von Figur 3 bzw. als Einrichtung 215 einer Vorrichtung 100 von Figur 2 zur Bestimmung eines neurologischen Gesundheitszustands einer Person 105 eingesetzt werden kann.

Das Verfahren 400 arbeitet bevorzugt mit einer Anzahl von Referenzpersonen, die sich jeweils im Wesentlichen wie die Person 105 verhalten, deren neurologischer Gesundheitszustand jedoch zusätzlich bekannt ist.

In einem Schritt 405 kann eine Referenzperson ausgewählt werden. Anschließend kann in einem Schritt 410 ein neurologischer Gesundheitszustand der Referenzperson erfasst werden. Dieser Schritt kann das Einholen oder Erstellen einer medizinischen Diagnose auf der Basis weiterer Prüfungen oder Verfahren umfassen.

In einem Schritt 415 kann ein optischer Reiz 115 erzeugt und der Person 105 über eine Anzeige 110 bereitgestellt werden. Alternativ kann ein ihr dargebotener optischer Reiz 115 erfasst und auf Verarbeitbarkeit überprüft werden (vgl. Schritt 315 des Verfahrens 300).

In einem Schritt 420 kann eine Augenbewegung der Person 105 bezüglich des optischen Reizes 115 erfasst werden. Auf dieser Basis kann die Blickrichtung 120 bezüglich des optischen Reizes 115 bestimmt werden. Die Blickrichtung 120 gibt üblicherweise einen Punkt auf der Anzeige 110 an, auf den die Person 105 gerade blickt. Im Fall voneinander unabhängiger Abtastungen von Blickrichtungen 120 beider Augen der Person 105 können auch Koordinaten zweier Punkte bestimmt werden. Üblicherweise erfolgt eine Betrachtung über eine gewisse Zeit hinweg, wobei auch eine Bewegungsgeschwindigkeit der Blickrichtung 120 bestimmt werden kann.

In einem Schritt 425 können Trainingsdaten gebildet werden, welche den optischen Reiz 115, die bestimmte Blickrichtung 120 sowie einen Hinweis auf den neurologischen Gesundheitszustand der Referenzperson umfassen.

In einem Schritt 430 kann bestimmt werden, ob von der Referenzperson ausreichend Daten erhoben worden sind. Ist dies nicht der Fall, so können die Schritte 415 bis 430 erneut durchlaufen werden. Andernfalls kann das Verfahren 400 zum Schritt 405 verzweigen, und es kann eine neue Referenzperson gewählt werden, mit der die beschriebenen, nachfolgenden Schritte ausgeführt werden.

Die im Schritt 425 gesammelten Daten können zu einem beliebigen Zeitpunkt in einem Schritt 435 dazu verwendet werden, eine Einrichtung 215 darauf zu trainieren, einen auffälligen von einem unauffälligen neurologischen Gesundheitszustand einer Person 105 zu unterscheiden. In einer Ausführungsform werden unterschiedliche Merkmale der Blickrichtung 120 bezüglich des optischen Reizes 115 bestimmt. Jedes Merkmal kann in einer gewissen Ausprägung vorliegen.

In einer ersten Variante wird im Schritt 435 ein Entscheidungsbaum darauf trainiert, die genannte Unterscheidung durchzuführen. In einer zweiten Variante werden mehrere Entscheidungsbäume nach Art eines Zufallswalds erzeugt und trainiert. Das Training kann beendet werden, wenn eine Erkennungsleistung der Einrichtung 215 bezüglich eines neurologischen Gesundheitszustands einer der Referenzpersonen ausreichend hoch ist.

Wie durch unterbrochene Linien angedeutet ist, kann ein Ergebnis des Trainierens im Schritt 435 des zweiten Verfahrens 400 im Schritt 330 des ersten Verfahrens 300 eingesetzt werden. Die trainierte Einrichtung 215 kann auch in der Vorrichtung 100 von Figur 2 oder in der Verarbeitungseinrichtung 135 von Figur 1 eingesetzt werden.

Unabhängig vom grammatikalischen Geschlecht eines bestimmten Begriffes sind hierin erwähnte Personen mit männlicher, weiblicher oder anderer Geschlechteridentität stets mit umfasst.

### Bezugszeichenliste (als Teil der Beschreibung)

- 100: Vorrichtung
- 105: Personen
- 110: Anzeige
- 115: optischer Reiz
- 120: Blickrichtung
- 125: Abtasteinrichtung
- 130: Computer
- 135: Verarbeitungseinrichtung

- 200: System
- 205: erste Schnittstelle
- 210: zweite Schnittstelle
- 215: Einrichtung zum maschinellen Lernen
- 220: Datenspeicher
- 225: dritte Schnittstelle
- 230: externe Einrichtung

- 300: Verfahren
- 305: Benutzereinverständnis einholen, Funktion anzeigen
- 310: optischen Reiz erfassen
- 315: verarbeitbarer Reiz?
- 320: Augenbewegung erfassen
- 325: neurologisches Merkmal bestimmen
- 330: neurologischen Gesundheitszustand bestimmen
- 335: Hinweis auf Bestimmungsergebnis bereitstellen

- 400: Verfahren
- 405: Referenzperson wählen
- 410: neurologischen Gesundheitszustand Referenzperson erfassen
- 415: optischen Reiz erzeugen oder verarbeitbaren Reiz erfassen
- 420: Augenbewegung erfassen
- 425: Trainingsdaten erfassen
- 430: Training zu Referenzperson abgeschlossen?
- 435: Trainieren

## Patentansprüche

1. Vorrichtung (100) zur Bestimmung eines neurologischen Gesundheitszustands einer Person (105), wobei die Vorrichtung (100) folgende Elemente umfasst:
- eine optische Anzeige (110) zur Bereitstellung eines optischen Reizes (115) an die Person (105);
- eine Abtasteinrichtung (125) zur Bestimmung einer Blickrichtung (120) der Person (105) bezüglich des optischen Reizes (115);
- eine Verarbeitungseinrichtung (135), die dazu eingerichtet ist, auf der Basis der erkannten Blickrichtung (120) und eines bereitgestellten optischen Reizes (115) eine neurologische Schwäche der Person (105) zu erkennen; und
- eine Ausgabevorrichtung (110) zur Bereitstellung eines Bestimmungsergebnisses.

2. Vorrichtung (100) nach Anspruch 1, wobei die Verarbeitungseinrichtung (135) dazu eingerichtet ist, mehrere vorbestimmte Merkmale der Blickrichtung (120) bezüglich des optischen Reizes (115) zu bestimmten.

3. Vorrichtung (100) nach Anspruch 1 oder 2, wobei der optische Reiz (115) aus einer externen Quelle (130) stammt und die Verarbeitungseinrichtung (135) dazu eingerichtet ist, den optischen Reiz (115) zu erfassen.

4. Vorrichtung (100) nach Anspruch 3, wobei die Verarbeitungseinrichtung (135) dazu eingerichtet ist, den externen optischen Reiz (115) zu klassifizieren und die Bestimmung durchzuführen, wenn der Reiz (115) in eine vorbestimmte Klasse fällt.

5. Vorrichtung (100) nach Anspruch 3 oder 4, wobei die Anzeige (110) einen Bildschirm und der optische Reiz (115) eine Ausgabe einer vorbestimmten Computeranwendung umfasst.

6. Vorrichtung (100) nach einem der vorangehenden Ansprüche, wobei die Verarbeitungseinrichtung (135) eine Methode des maschinellen Lernens (215) realisiert und darauf trainiert ist, auf der Basis der erkannten Blickrichtung (120) und des bereitgestellten optischen Reizes (115) eine vorbestimmte neurologische Schwäche zu erkennen.

7. Vorrichtung (100) nach Anspruch 6, wobei die Methode des maschinellen Lernens einen Entscheidungsbaum (215) umfasst.

8. Vorrichtung (100) nach Anspruch 6, wobei die Methode des maschinellen Lernens einen Zufallswald (215) umfasst.

9. Vorrichtung (100) nach einem der vorangehenden Ansprüche, wobei die Abtasteinrichtung (125) eine Kamera zur Abtastung eines Auges der Person (105) umfasst.

10. Vorrichtung (100) nach einem der vorangehenden Ansprüche, ferner umfassend eine Ausgabeeinrichtung (110) an die Person (105), wobei die Vorrichtung (100) dazu eingerichtet ist, einen Hinweis auf eine Bestimmung des Gesundheitszustands bereitzustellen.

11. Vorrichtung (100) nach einem der vorangehenden Ansprüche, ferner umfassend eine Eingabeeinrichtung für die Person (105), wobei die Verarbeitungseinrichtung (135) dazu eingerichtet ist, ein Einverständnis der Person (105) zur Durchführung einer Bestimmung ihres Gesundheitszustands zu erfassen.

12. Verfahren (300) zum Bestimmen eines neurologischen Gesundheitszustands einer Person (105), wobei das Verfahren folgende Schritte umfasst:
- Erfassen (310) eines der Person (105) dargebotenen optischen Reizes (115);
- Bestimmen (320) einer Blickrichtung (120) der Person (105) bezüglich des optischen Reizes (115);
- Erkennen (330) einer neurologische Schwäche der Person (105) auf der Basis der erkannten Blickrichtung (120) und des bereitgestellten optischen Reizes (115); und
- Bereitstellen (335) eines Bestimmungsergebnisses.

13. Verfahren (400) zum Trainieren einer Verarbeitungseinrichtung (135), die eine Technik des maschinellen Lernens realisiert, zur Bestimmung eines neurologischen Gesundheitszustands einer Person (105), wobei das Verfahren folgende Schritte umfasst:
- Erfassen (410) eines neurologischen Gesundheitszustands einer Referenzperson (105);
- Verfolgen (420) einer Blickrichtung (120) der Referenzperson (105) auf einer Anzeige (110), auf welcher der Referenzperson (105) ein vorbestimmter optischer Reiz (115) dargeboten ist;
- Trainieren (430) der Verarbeitungseinrichtung (135) zur Erkennung des erfassten neurologischen Gesundheitszustands auf das Basis der Blickrichtung (120);
- wobei die genannten Schritte an einer Vielzahl Referenzpersonen (105) durchgeführt werden.

14. Verfahren (400) nach Anspruch 13, die Referenzpersonen erste Personen (105) umfassen, die neurologisch unauffällig sind, und zweite Personen (105), die an einer neurologischen Schwäche leiden.
